# EUROPEAN PATENT SPECIFICATION

(11) **EP 2 553 085 B1**
(45) Date of publication and mention of the grant of the patent: **28.04.2021**
(21) Application number: 11760338.1
(22) Date of filing: 25.03.2011
(51) Int. Cl.: C12M 1/38, C12Q 1/68, B01L 3/00, B01L 7/00

(54) **DEVICES, SYSTEMS, AND METHODS FOR AMPLIFYING NUCLEIC ACIDS**
VORRICHTUNGEN, SYSTEME UND VERFAHREN ZUR NUKLEINSÄUREAMPLIFIKATION
DISPOSITIFS, SYSTÈMES, ET PROCÉDÉS POUR AMPLIFIER DES ACIDES NUCLÉIQUES

(30) Priority: 26.03.2010 US 732596
(43) Date of publication of application: 06.02.2013
(73) Proprietor: Stokes Bio Limited, Limerick (IE)
(72) Inventor: DAVIES, Mark, Limerick (IE); DALTON, Tara, Limerick (IE)
(74) Representative: Ziermann, Oliver
(86) International application number: PCT/US2011/030047
(87) International publication number: WO 2011/119994

(56) References cited:
- EP-A2- 1 584 692
- WO-A1-03/016558
- WO-A1-2005/002730
- WO-A1-2007/091228
- WO-A1-2007/091230
- WO-A2-2006/089192
- US-A1- 2001 042 712
- US-A1- 2005 186 585
- US-A1- 2005 221 373
- US-A1- 2006 127 889
- US-A1- 2008 280 331
- US-A1- 2009 317 874
- US-A1- 2009 317 874
- KOPP ET AL: "chemical amplification:continuous flow PCR on a chip", SCIENCE, AMERICAN ASSOCIATION FOR THE ADVANCEMENT OF SCIENCE, WASHINGTON, DC; US, vol. 280, 15 May 1998 (1998-05-15), pages 1046-1048, XP002107956, ISSN: 0036-8075, DOI: 10.1126/SCIENCE.280.5366.1046

## Description

### Field of the Invention

The present invention generally relates to a devices, systems, and methods for amplifying nucleic acids in flowing droplets.

### Background

Microfluidics involves micro-scale devices that handle small volumes of fluids. Because microfluidics can accurately and reproducibly control small fluid volumes, in particular volumes less than 1 µl, application of microfluidics provides significant cost-savings. The use of microfluidics technology reduces cycle times, shortens time-to-results, and increases throughput. Furthermore incorporation of microfluidics technology enhances system integration and automation.

An exemplary microfluidic device involves liquid bridge technology. Liquid bridges allow sample droplet formation or mixing utilizing immiscible fluids. In a liquid bridge, a sample droplet at an end of an inlet port enters a chamber that is filled with a carrier fluid. The carrier fluid is immiscible with the sample droplet. The sample droplet expands until it is large enough to span a gap between inlet and outlet ports. Droplet mixing can be accomplished in many ways, for example, by adjusting flow rate or by introducing a second sample droplet to the first sample droplet, forming an unstable funicular bridge that subsequently ruptures from the inlet port. After rupturing from the inlet port, the mixed sample droplet enters the outlet port, surrounded by the carrier fluid from the chamber. WO 2007/091230 A1, US 2009/317874 A1, WO 2006/089192 A2 and EP 1 584 692 A2 show examples of microfluidic analysis systems.

### Summary

Liquid bridges may be connected to thermocyclers in order to amplify nucleic acids within sample droplets. Of particular usefulness, is the ability to perform quantitative polymerase chain reaction (QPCR) on nucleic acids in flowing mixed sample droplets generated in a liquid bridge system. QPCR is a technique based on the polymerase chain reaction, which is used to amplify and simultaneously quantify a targeted nucleic acid. An important feature of QPCR is that the amplified nucleic acid is detected as the reaction progresses in real-time. Thus sufficient optical access to a sample is required so that amplification of nucleic acids in the sample may be assessed in real-time, i.e., after each amplification cycle.

The present invention generally relates to devices, systems, and methods that provide sufficient optical access to flowing mixed sample droplets, such as those generated in a liquid bridge system, so that amplification of nucleic acids in the sample droplets may be assessed in real-time, i.e., after each amplification cycle. Aspects of the invention are accomplished by positioning at least one channel in thermal contact with a plurality of temperature zones in such a configuration that optical access to the droplets in the channel is maintained as the droplets flow through the channel. Because the channel is in thermal contact with the plurality of temperature zones, nucleic acids in the droplets may undergo a polymerase chain reaction while flowing through the channel. Because the channel is positioned with respect to the temperature zones so that the channel provides optical access to the nucleic acids being amplified within the sample droplets as they flow through the channel, optical detection of nucleic acid amplification in real-time may be achieved, and QPCR may be performed on the nucleic acids in the flowing sample droplets.

In order to perform a polymerase chain reaction on the nucleic acids in the flowing droplets, the temperature zones are at different locations along the channel and the zones are separated from each other. In particular embodiments, air gaps are used to separate the temperature zones. The plurality of temperature zones may include a first temperature zone sufficient to provide a temperature that results in denaturation of double stranded nucleic acids to produce single stranded nucleic acids, a second temperature zone sufficient to provide a temperature that results in hybridization of primers to the single stranded nucleic acids, and a third temperature zone sufficient to provide a temperature that results in amplification of single stranded nucleic acids to produce double stranded nucleic acids. The plurality of temperature zones may be arranged as repeating first, second, and third zones.

Devices and systems of the invention may also be configured such that the channel containing the flowing droplets lies within a second channel (i.e., a first channel lying within a second channel). In certain embodiments, the second channel has an open top so that optical access to the flowing droplets in the first channel is maintained. In a particular embodiment, the droplets are wrapped in an immiscible fluid. Devices and systems of the invention may also be coupled to an illumination system and an optical detection device.

Another aspect of the invention provides a method for performing a quantitative polymerase chain reaction including the steps of: a) flowing sample droplets comprising nucleic acids through at least one first channel, in which the nucleic acids in the droplets are optically detectable while the droplets are flowing through the first channel, b) denaturing double stranded nucleic acids in the flowing sample droplets to produce single stranded nucleic acids, c) hybridizing primers to the single stranded nucleic acids in the flowing sample droplets, d) simultaneously amplifying the nucleic acids and detecting the amplified nucleic acids in the flowing droplets, and e) repeating steps (b) through (d) at least once. In certain embodiments, three temperature zones are used, while in other embodiments, two temperature zones are used. In a particular embodiment, mixed droplets are wrapped in an immiscible carrier fluid, and the wrapped mixed droplets are generated from a liquid bridge system.

### Brief Description of the Drawings

Figure 1 is a schematic showing an exemplary embodiment of a device of the invention.
Figure 2 is a schematic showing an exemplary embodiment of a configuration of a two channel device.
Figure 3 is a graph showing an example of a fluorescence amplification curve.

### Detailed Description

The present invention generally relates to a devices, and methods for amplifying nucleic acids in flowing droplets according to claims 1-13. Amplification refers to production of additional copies of a nucleic acid sequence and is generally carried out using polymerase chain reaction or other technologies well known in the art (e.g., Dieffenbach and Dveksler, PCR Primer, a Laboratory Manual, Cold Spring Harbor Press, Plainview, N.Y., 1995). Polymerase chain reaction (PCR) refers to methods by K. B. Mullis (U.S. patent numbers 4,683,195 and 4,683,202, hereby incorporated by reference) for increasing concentration of a segment of a target sequence in a mixture of genomic DNA without cloning or purification. The process for amplifying the target sequence includes introducing an excess of oligonucleotide primers to a DNA mixture containing a desired target sequence, followed by a precise sequence of thermal cycling (heating and cooling) in the presence of a DNA polymerase. The primers are complementary to their respective strands of the double stranded target sequence.

To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one cycle; there can be numerous cycles) to obtain a high concentration of an amplified segment of a desired target sequence. The length of the amplified segment of the desired target sequence is determined by relative positions of the primers with respect to each other, and therefore, this length is a controllable parameter.

In a particular embodiment, the amplification reaction is a quantitative amplification reaction. QPCR is a technique based on the polymerase chain reaction, and is used to amplify and simultaneously quantify a targeted nucleic acid molecule. QPCR allows for both detection and quantification (as absolute number of copies or relative amount when normalized to DNA input or additional normalizing genes) of a specific sequence in a DNA sample. The procedure follows the general principle of polymerase chain reaction, with the additional feature that the amplified DNA is quantified as it accumulates in the reaction in real-time after each amplification cycle. QPCR is described, for example, in Kurnit et al. (U.S. patent number 6,033,854), Wang et al. (U.S. patent number 5,567,583 and 5,348,853), Ma et al. (The Journal of American Science, 2(3), 2006), Heid et al. (Genome Research 986-994, 1996), Sambrook and Russell (Quantitative PCR, Cold Spring Harbor Protocols, 2006), and Higuchi (U.S. patent numbers 6,171,785 and 5,994,056).

Two common methods of quantification are: (1) use of fluorescent dyes that intercalate with double-stranded DNA, and (2) modified DNA oligonucleotide probes that fluoresce when hybridized with a complementary DNA. In the first method, a DNA-binding dye binds to all double-stranded (ds)DNA in PCR, resulting in fluorescence of the dye. An increase in DNA product during PCR therefore leads to an increase in fluorescence intensity and is measured at each cycle, thus allowing DNA concentrations to be quantified. The reaction is prepared similarly to a standard PCR reaction, with the addition of fluorescent (ds)DNA dye. The reaction is run in a thermocycler, and after each cycle, the levels of fluorescence are measured with a detector; the dye only fluoresces when bound to the (ds)DNA (i.e., the PCR product). With reference to a standard dilution, the (ds)DNA concentration in the PCR can be determined. Like other real-time PCR methods, the values obtained do not have absolute units associated with it. A comparison of a measured DNA/RNA sample to a standard dilution gives a fraction or ratio of the sample relative to the standard, allowing relative comparisons between different tissues or experimental conditions. To ensure accuracy in the quantification, it is important to normalize expression of a target gene to a stably expressed gene. This allows for correction of possible differences in nucleic acid quantity or quality across samples.

The second method uses a sequence-specific RNA or DNA-based probe to quantify only the DNA containing the probe sequence; therefore, use of the reporter probe significantly increases specificity, and allows quantification even in the presence of some non-specific DNA amplification. This allows for multiplexing, i.e., assaying for several genes in the same reaction by using specific probes with differently colored labels, provided that all genes are amplified with similar efficiency.

This method is commonly carried out with a DNA-based probe with a fluorescent reporter (e.g. 6-carboxyfluorescein) at one end and a quencher (e.g., 6-carboxy-tetramethylrhodamine) of fluorescence at the opposite end of the probe. The close proximity of the reporter to the quencher prevents detection of its fluorescence. Breakdown of the probe by the 5' to 3' exonuclease activity of a polymerase (e.g., Taq polymerase) breaks the reporter-quencher proximity and thus allows unquenched emission of fluorescence, which can be detected. An increase in the product targeted by the reporter probe at each PCR cycle results in a proportional increase in fluorescence due to breakdown of the probe and release of the reporter. The reaction is prepared similarly to a standard PCR reaction, and the reporter probe is added. As the reaction commences, during the annealing stage of the PCR both probe and primers anneal to the DNA target. Polymerization of a new DNA strand is initiated from the primers, and once the polymerase reaches the probe, its 5'-3'-exonuclease degrades the probe, physically separating the fluorescent reporter from the quencher, resulting in an increase in fluorescence. Fluorescence is detected and measured in a real-time PCR thermocycler, and geometric increase of fluorescence corresponding to exponential increase of the product is used to determine the threshold cycle in each reaction.

Relative concentrations of DNA present during the exponential phase of the reaction are determined by plotting fluorescence against cycle number on a logarithmic scale (so an exponentially increasing quantity will give a straight line). A threshold for detection of fluorescence above background is determined. The cycle at which the fluorescence from a sample crosses the threshold is called the cycle threshold, Cₜ. Since the quantity of DNA doubles every cycle during the exponential phase, relative amounts of DNA can be calculated, e.g. a sample with a Cₜ of 3 cycles earlier than another has 2³ = 8 times more template. Amounts of nucleic acid (e.g., RNA or DNA) are then determined by comparing the results to a standard curve produced by a real-time PCR of serial dilutions (e.g. undiluted, 1:4, 1:16, 1:64) of a known amount of nucleic acid.

In certain embodiments, the QPCR reaction involves a dual fluorophore approach that takes advantage of fluorescence resonance energy transfer (FRET), e.g., LIGHTCYCLER hybridization probes, where two oligonucleotide probes anneal to the amplicon (e.g. see U.S. patent number 6,174,670). The oligonucleotides are designed to hybridize in a head-to-tail orientation with the fluorophores separated at a distance that is compatible with efficient energy transfer. Other examples of labeled oligonucleotides that are structured to emit a signal when bound to a nucleic acid or incorporated into an extension product include: SCORPIONS probes (e.g., Whitcombe et al., Nature Biotechnology 17:804-807, 1999, and U.S. patent number 6,326,145), Sunrise (or AMPLIFLOUR) primers (e.g., Nazarenko et al., Nuc. Acids Res. 25:2516-2521, 1997, and U.S. patent number 6,117,635), and LUX primers and MOLECULAR BEACONS probes (e.g., Tyagi et al., Nature Biotechnology 14:303-308, 1996 and U.S. patent number 5,989,823).

In other embodiments, the QPCR reaction uses fluorescent Taqman methodology and an instrument capable of measuring fluorescence in real-time (e.g., ABI Prism 7700 Sequence Detector). The Taqman reaction uses a hybridization probe labeled with two different fluorescent dyes. One dye is a reporter dye (6-carboxyfluorescein), the other is a quenching dye (6-carboxy-tetramethylrhodamine). When the probe is intact, fluorescent energy transfer occurs and the reporter dye fluorescent emission is absorbed by the quenching dye. During the extension phase of the PCR cycle, the fluorescent hybridization probe is cleaved by the 5'-3' nucleolytic activity of the DNA polymerase. On cleavage of the probe, the reporter dye emission is no longer transferred efficiently to the quenching dye, resulting in an increase of the reporter dye fluorescent emission spectra.

In order to perform QPCR on nucleic acids in flowing mixed sample droplets generated from a liquid bridge system, sufficient optical access to the nucleic acids in the droplets is required. The present invention generally relates to devices, systems, and methods that provide sufficient optical access to flowing mixed sample droplets generated from a liquid bridge system so that amplification of nucleic acids in the sample droplets may be assessed in real-time, i.e., after each amplification cycle. Aspects of the invention provide devices for amplifying nucleic acids including at least one first channel through which sample droplets including nucleic acids flow, in which the nucleic acids in the droplets are optically detectable while the droplets are flowing through the first channel, and a plurality of temperature zones in thermal contact with the first channel, in which the zones are located at different locations along the first channel and the zones are separated from each other.

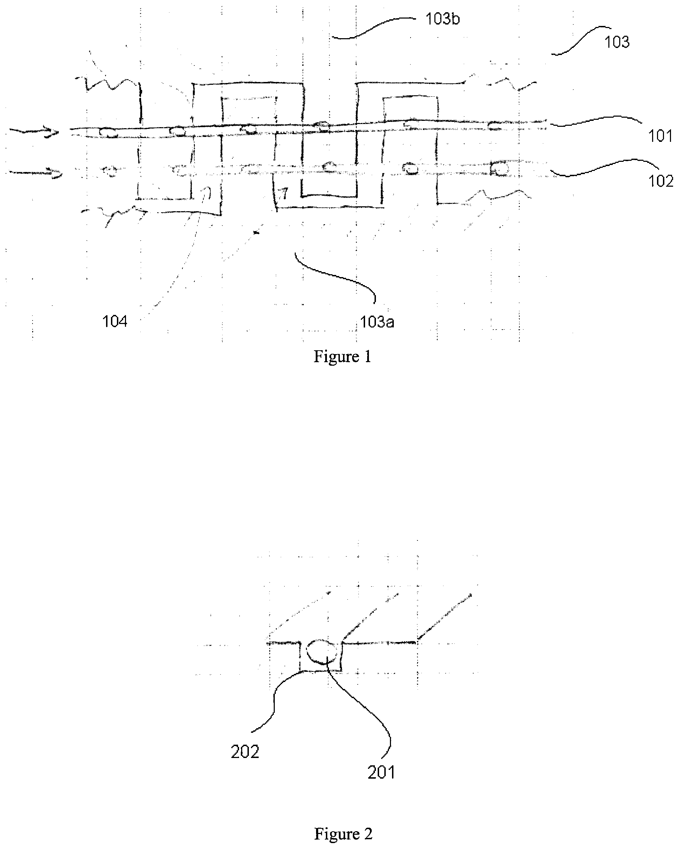

Figure 1 provides an exemplary embodiment of a device 100 of the invention. Device **100** includes first channels **101** and **102** through which sample droplets flow. In this embodiment, the channels are positioned over a plurality of temperature zones **103.** In this embodiment, a high temperature zone **103a** and a low temperature zone **103b** is shown. High and low temperature zones **103a** and **103b** are thermally isolated from each other by air gaps **104.**

Figure 1 provides an embodiment of the device showing straight channels; however, many different channel designs are possible. For example, embodiments of the device may include curved channels. In other embodiments, the device is designed such that the amount of curve or the number of curves in the channel varies depending on the temperature zone. In still other embodiments, the channel is a wrap around channel, such that droplets in a single channel pass through the same temperature zones more than once. Utilizing a wrap around channel eliminates the need for providing additional temperature zones and allows for a single set of temperature zones to be used multiple times.

Devices and systems of the invention may also be configured such that the first channel containing the flowing droplets lies within a second channel (i.e., a first channel lying within a second channel). See Figure 2, which shows an exemplary configuration of channel **201** laying within channel **202.** The second channel is configured such that at least one side of the second channel provides optical access to droplets flowing through the first channel. For example, in certain embodiments, the second channel has an open top so that optical access to the flowing droplets in the first channel is maintained. Alternatively, the top of the second channel is made of a transparent material so that optical access to the flowing droplets in the first channel is not impeded. In other embodiments, a bottom or one of the sides of the second channel is made of a transparent material so that optical access to the flowing droplets in the first channel is not impeded. In other embodiments, the first channel is enclosed by the second channel, and the second channel is made of a transparent material so that optical access to the flowing droplets in the first channel is not impeded.

The first channel may be made of any material suitable to interact with biological or chemical species and that allows for optical detection of nucleic acids within droplets that are flowing through the channel. Exemplary materials include TEFLON (commercially available from Dupont, Wilmington, DE), polytetrafluoroethylene (PTFE; commercially available from Dupont, Wilmington, DE), polymethyl methacrylate (PMMA; commercially available from TexLoc, Fort Worth, TX), polyurethane (commercially available from TexLoc, Fort Worth, TX), polycarbonate (commercially available from TexLoc, Fort Worth, TX), polystyrene (commercially available from TexLoc, Fort Worth, TX), polyetheretherketone (PEEK; commercially available from TexLoc, Fort Worth, TX), perfluoroalkoxy (PFA; commercially available from TexLoc, Fort Worth, TX), or Fluorinated ethylene propylene (FEP; commercially available from TexLoc, Fort Worth, TX). In particular embodiments, the first channel is made from PTFE. The second channel may be composed of the same or different material as the first channel.

The first channel contains flowing droplets including nucleic acids. Sample droplets are formed by a droplet forming device. Any device may be used that results in forming of sample droplets that are wrapped in an immiscible carrier fluid. Determination of the immiscible fluid to be used is based on the properties of the channel and of the sample. If the sample is a hydrophilic sample, the fluid used should be a hydrophobic fluid. An exemplary hydrophobic fluid is oil, such as AS5 silicone oil (commercially available from Union Carbide Corporation, Danbury, CN). Alternatively, if the sample is a hydrophobic sample, the fluid to used should be a hydrophilic fluid. One of skill in the art will readily be able to determine the type of fluid to be used based on the properties of the sample.

The wrapped droplets may be formed, for example, by dipping an open ended tube into a vessel. Exemplary sample acquisition devices are shown in McGuire et al. (U.S. patent application serial number 12/468,367). Alternatively, droplets may be formed by flowing a continuous plug of sample to a liquid bridge and using the liquid bridge to form the droplets.

After droplet formation, sample droplets are mixed by a droplet mixing device. The droplet mixing device may be any device that is capable of mixing sample droplets to form mixed sample droplets wrapped in an immiscible carrier fluid. An exemplary droplet mixing device is a liquid bridge. Liquid bridges allow sample droplet mixing utilizing immiscible fluids. In a liquid bridge, a sample droplet at an end of an inlet port enters a chamber that is filled with a carrier fluid. The carrier fluid is immiscible with the sample droplet. The sample droplet expands until it is large enough to span a gap between inlet and outlet ports. Droplet mixing can be accomplished in many ways, for example, by adjusting flow rate or by introducing a second sample droplet to the first sample droplet, forming an unstable funicular bridge that subsequently ruptures from the inlet port. After rupturing from the inlet port, the mixed sample droplet enters the outlet port, surrounded by the carrier fluid from the chamber. An exemplary liquid bridge system is shown in Davies et al. (International patent publication number WO 2007/091228), the contents of which are incorporated by reference herein in their entirety.

After droplet mixing, the wrapped mixed samples droplets continue to flow through the channel to undergo amplification. The first channel is in thermal contact with a plurality of temperature zones, and the channel is configured with respect to the temperature zones so that optical access to droplets in the channel is maintained. Optical access to droplets flowing through the first channel may be achieved in numerous ways, for example, by configuring the devices of the invention such that the first channel is positioned over the temperature zones, or by configuring the devices of the invention such that the first channel is positioned such that the temperature zones on located on both sides of the channel. Any configuration of the first channel with the temperature zones that results in optical access to the sample droplets flowing through the first channel is envisioned herein. In particular embodiments, the first channel is configured to lay over the temperature zones, providing optical access to the droplets within the first channel.

In certain embodiments, the temperature zones are controlled to achieve three individual temperature zones for a PCR reaction. Each temperature zone is controlled by continuous temperature sensing and a PID feedback control system. The temperature zones are separated from each other using air gaps. The air gaps may be of any distance to provide thermal isolation of the temperature zones and still allow for a PCR reaction to be conducted on the nucleic acids within the flowing droplets. The air gap may range from about 1 mm to about 10 cm. In particular embodiments, the air gap is approximately 1 mm.

Each temperature zone is controlled to be isothermal with respect to time. Residency time of the droplets in each temperature zone is determined by the relationship of the length of the channel to the velocity of the droplet moving through the channel. By increasing channel length in each temperature zone, e.g., by curving the channel, without varying velocity of the droplets moving through the channel, the residency time of the droplets in the temperature increases. In certain embodiments, the channel length in each temperature zone is different, and thus droplets will spend different amounts of time in different temperature zones. For example, in certain embodiments the residency time of droplets in the 90° temperature zone is different than the residency time of droplets in the 65° temperature zone.

Velocity of the droplets through the devices of the invention is controlled by the velocity of the carrier fluid. In certain embodiments, flow is controlled by a pump. In other embodiments, the flow is controlled by a siphoning effect, generated based on the configuration of the system. See co-pending application serial number 12/683,882 by Davies et al., filed with the U.S. Patent and Trademark Office on January 7, 2010, and entitled "Sample Dispensing". The velocity may then be varied to control residency time of the droplet in each temperature zone.

The above relationship may be used to design different size thermocyclers that produce the same residency time of droplets in each temperature zone by varying either the channel length in each temperature zone, the velocity of the droplets, or both accordingly.

In certain embodiments, devices of the invention include interchangeable components allowing for devices of the invention to be re-configurable. Each interchangeable component includes a geometry that produces a different channel length for the channel in that component. As discussed above, varying the channel length in a temperature zone will vary the residency time for a droplet in that temperature zone.

The three temperature zones are controlled to result in denaturation of double stranded nucleic acids (high temperature zone), annealing of primers (low temperature zones), and amplification of single stranded nucleic acid to produce double stranded nucleic acids (intermediate temperature zones). The temperatures within these zones fall within ranges well known in the art for conducting PCR reactions. See for example, Sambrook et al. (Molecular Cloning, A Laboratory Manual, 3rd edition, Cold Spring Harbor Laboratory Press, Cold Spring Harbor, New York, 2001).

In certain embodiments, the three temperature zones are controlled to have temperatures as follows: 95°C (T_{H}), 55°C (T_{L}), 72°C (T_{M}). The prepared sample droplets, wrapped in the carrier fluid, flow through the channel at a controlled rate. The sample droplets first pass the initial denaturation zone (T_{H}) before thermal cycling. The initial preheat is an extended zone to ensure that nucleic acids within the sample droplet have denatured successfully before thermal cycling. The requirement for a preheat zone and the length of denaturation time required is dependent on the chemistry being used in the reaction. The samples pass into the high temperature zone, of approximately 95°C, where the sample is first separated into single stranded DNA in a process called denaturation. The sample then flows to the low temperature, of approximately 55°C, where the hybridization process takes place, during which the primers anneal to the complementary sequences of the sample. Finally, as the sample flows through the third medium temperature, of approximately 72°C, the polymerase process occurs when the primers are extended along the single strand of DNA with a thermostable enzyme.

The nucleic acids undergo the same thermal cycling and chemical reaction as the droplets passes through each thermal cycle as they flow through the channel. The total number of cycles in the device is easily altered by an extension of thermal zones. The sample undergoes the same thermal cycling and chemical reaction as it passes through N amplification cycles of the complete thermal device. This results in a maximum two-fold amplification after each cycle and a total amplification of I(1+E)^{N} where I is the initial product, E is the efficiency of the reaction and N is the number of cycles.

In other embodiments, the temperature zones are controlled to achieve two individual temperature zones for a PCR reaction. Each temperature zone is controlled by continuous temperature sensing and a PID feedback control system. The temperature zone are thermally separated from each other using air gaps. The air gaps may be of any distance to provide thermal isolation of the temperature zones and still allow for a PCR reaction to be conducted on the nucleic acids within the flowing droplets. The air gap may range from about 1 mm to about 10 cm. In particular embodiments, the air gap is approximately 1 mm.

Each temperature zone is controlled to be isothermal with respect to time. Velocity of the droplets through the devices of the invention is controlled by the velocity of the carrier fluid. In certain embodiments, flow is controlled by a pump. In other embodiments, the flow is controlled by a siphoning effect, generated based on the configuration of the system. See co-pending application serial number 12/683,882 by Davies et al., filed with the U.S. Patent and Trademark Office on January 7, 2010, and entitled "Sample Dispensing". The velocity may then be varied to control residency time of the droplet in each temperature zone.

In certain embodiments, the two temperature zones are controlled to have temperatures as follows: 95°C (T_{H}) and 60°C (T_{L}). The sample droplet optionally flows through an initial preheat zone before entering thermal cycling. The preheat zone may be important for some chemistry for activation and also to ensure that double stranded nucleic acids in the droplets are fully denatured before the thermal cycling reaction begins. In an exemplary embodiment, the preheat dwell length results in approximately 10 minutes preheat of the droplets at the higher temperature.

The sample droplet continues into the high temperature zone, of approximately 95°C, where the sample is first separated into single stranded DNA in a process called denaturation. The sample then flows through the device to the low temperature zone, of approximately 60°C, where the hybridization process takes place, during which the primers anneal to the complementary sequences of the sample. Finally the polymerase process occurs when the primers are extended along the single strand of DNA with a thermostable enzyme. The sample undergoes the same thermal cycling and chemical reaction as it passes through each thermal cycle of the complete device. The total number of cycles in the device is easily altered by an extension of block length and tubing.

Because the channel provides optical access to the nucleic acids in the flowing droplets, the nucleic acids may be optically detected after each amplification cycle and QPCR may be performed on the nucleic acids in the droplets. In certain embodiments, the present QPCR methods use fluorescent probes to monitor the amplification process as it progresses. SYBR Green 1 dye is a dye commonly used for fluorescent detection of double-stranded DNA generated during PCR. The dye exhibits a peak excitation maximum at 497 nm and a peak emission maximum at 520 nm. Taqman probes may also be used which are a more target specific probe. The Taqman probes have different excitation and emission wavelengths, and one example is the FAM labeled probe which has a peak excitation of 488 nm and an emission of 520 nm.

Through the analysis of the cycle-to-cycle change in fluorescence signal important information regarding the nucleic acid sample may be obtained. This is done by illuminating the sample and detecting the resulting fluorescence after each amplification cycle. Different product concentration will demonstrate fluorescence amplification at difference cycle numbers.

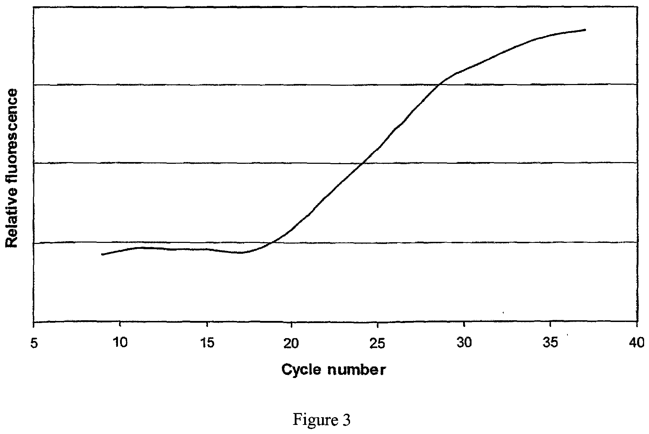

Figure 3 demonstrates an example of a fluorescence amplification curve. The curve is generated using a Taqman probe. There is little change in the fluorescent signal after the first number of thermal cycles. This defines the baseline for the amplification plot. Fluorescence intensity levels above this baseline represent amplification of PCR product. A fluorescent threshold can be fixed above this baseline that defines the threshold cycle, or Ct, for each reaction. The threshold cycle is defined as the fractional cycle number at which the fluorescence passes above a fixed threshold. Ct is observed in the early exponential stages of amplification. The higher the starting DNA template concentration, the sooner a significant increase in fluorescence is observed. Therefore the starting DNA concentration may be determined by the real-time fluorescent detection of the amplifying sample.

In order to perform a QPCR reaction, devices of the invention may be coupled to an excitation system for exciting the fluorescent probes and a detection system for detecting fluorescent emission from the probes after each amplification cycle. Excitation and detection systems of the invention allow for four-field excitation and four-field detection. Exemplary excitation and detection systems are shown in Davies et al. (International patent publication number WO 2007/091230, the contents of which are incorporated by reference herein in their entirety). An exemplary detection system may include detection system a light source; optics for focusing the incident light; filters for filtering the incident light; focusing optics for focusing fluorescence emitted by the sample; filter optics for filtering the emitted fluorescence; sensor electronics; and processing electronics. In certain embodiments, the detection system is a spectrograph.

Detection systems of the invention allow for detection of any type of optical label, such as a fluorescent label or dye. Detectable labels may be directly or indirectly detectable. Preferred labels include optically-detectable labels, such as fluorescent labels. Examples of fluorescent labels include, but are not limited to, 4-acetamido-4'-isothiocyanatostilbene-2,2'disulfonic acid; acridine and derivatives: acridine, acridine isothiocyanate; 5-(2'-aminoethyl)aminonaphthalene-1-sulfonic acid (EDANS); 4-amino-N-[3-vinylsulfonyl)phenyl]naphthalimide-3,5 disulfonate; N-(4-anilino-1-naphthyl)maleimide; anthranilamide; BODIPY; Brilliant Yellow; coumarin and derivatives; coumarin, 7-amino-4-methylcoumarin (AMC, Coumarin 120), 7-amino-4-trifluoromethylcouluarin (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethylamino-3 -(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron.TM. Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N',N'tetramethyl-6-carboxyrhodamine (TAMRA); tetramethyl rhodamine; tetramethyl rhodamine isothiocyanate (TRITC); riboflavin; rosolic acid; terbium chelate derivatives; Cy3; Cy5; Cy5.5; Cy7; IRD 700; IRD 800; La Jolta Blue; phthalo cyanine; and naphthalo cyanine. Labels other than fluorescent labels are contemplated by the invention, including other optically-detectable labels.

The choice of light source is dependent on the remainder of the detection system but there are many options including filtered white light, specific wavelength laser or laser diode. Fiber optics may also be incorporated for light transport. The filtering is dependent on the light source and detection system but commercially available filter components may be used.

If a detection indicator is used this will be provided in the sample preparation system. The detection sensor used is dependent on the field of view required and the illumination wavelength chosen. Detector options include CCD, CMOS, photodiode and photomultipliers.

## Claims

1. A device (100) for amplifying nucleic acids, the device (100) comprising:
at least one first channel (101) through which sample droplets comprising nucleic acids flow, wherein the nucleic acids in the droplets are optically detectable while the droplets are flowing through the first channel (101); and
a plurality of temperature zones comprising a high temperature zone (103a) and a low temperature zone (103b), each zone having a profile defined by rectangular projections alternating with rectangular recesses, each rectangular recess of the high temperature zone enclosing a corresponding rectangular projection of the low temperature zone and vice versa, the high temperature zone and the low temperature zone being thermally isolated from each other by air gaps (104), wherein the high temperature zone (103a) and the low temperature zone (103b) are in thermal contact with the first channel (101), which is configured in a straight line in the area of thermal contact with the high and the low temperature zone, wherein portions of the high temperature zone alternate with portions of the low temperature zone along the first channel (101) and the zones (103a, 103b) are separated from each other.

2. The device (100) according to claim 1, wherein a first temperature zone is suitable to provide a temperature sufficient to result in denaturation of double stranded nucleic acids to produce single stranded nucleic acids, a second temperature zone is suitable to provide a temperature sufficient to result in hybridization of primers to the single stranded nucleic acids, and a third temperature zone is suitable to provide a temperature sufficient to result in amplification of single stranded nucleic acids to produce double stranded nucleic acids.

3. The device (100) according to claim 1, further comprising a second channel, wherein the second channel is open at a top and the first channel (101) lies within the second channel.

4. The device (100) according to claim 1, wherein the device (100) is coupled to an excitation system.

5. The device (100) according to claim 4, wherein the device (100) is coupled to an optical detection device.

6. The device (100) according to claim 5, wherein the optical detection device is a spectrograph.

7. The device (100) according to claim 1, wherein the device is fluidically coupled to a liquid bridge system.

8. The device (100) according to claim 1, wherein the droplets are wrapped in an immiscible fluid.

9. The device (100) according to claim 1, wherein the nucleic acid is DNA or RNA.

10. A method for performing a quantitative polymerase chain reaction using a device (100) according to any of the claims 1-9, the method comprising the steps of:
a) flowing sample droplets comprising labeled nucleic acids through the at least one first channel (101), wherein the labeled nucleic acids in the droplets are optically detectable while the droplets are flowing through the first channel (101);
b) denaturing double stranded nucleic acids in the flowing sample droplets to produce single stranded nucleic acids;
c) hybridizing primers to the single stranded nucleic acids in the flowing sample droplets;
d) simultaneously amplifying the nucleic acids and detecting the amplified nucleic acids in the flowing droplets; and
e) repeating steps (b) through (d) at least once.

11. The method according to claim 10, wherein the droplets are wrapped in an immiscible fluid.

12. The method according to claim 10, wherein prior to step (a), the method further comprises forming sample droplets using a liquid bridge system.

13. The method according to claim 10, wherein the first channel (101) lies within a second channel open at a top.

## Patentansprüche

1. Vorrichtung (100) zum Amplifizieren von Nukleinsäuren, wobei die Vorrichtung (100) Folgendes umfasst:
wenigstens einen ersten Kanal (101), durch den Probentröpfchen fließen, die Nukleinsäuren umfassen, wobei die Nukleinsäuren in den Tröpfchen optisch erfassbar sind, während die Tröpfchen durch den ersten Kanal (101) fließen; und
mehrere Temperaturbereiche, die einen Hochtemperaturbereich (103a) und einen Niedertemperaturbereich (103b) umfassen, wobei jeder Bereich ein Profil aufweist, das durch rechteckige Vorsprünge definiert ist, die sich mit rechteckigen Aussparungen abwechseln, wobei jede rechteckige Aussparung des Hochtemperaturbereichs einen entsprechenden rechteckigen Vorsprung des Niedertemperaturbereichs umschließt und umgekehrt, wobei der Hochtemperaturbereich und der Niedertemperaturbereich durch Luftspalte (104) thermisch voneinander isoliert sind, wobei der Hochtemperaturbereich (103a) und der Niedertemperaturbereich (103b) in thermischem Kontakt mit dem ersten Kanal (101) stehen, der in dem Gebiet des thermischen Kontakts mit dem Hoch- und dem Niedertemperaturbereich geradlinig konfiguriert ist, wobei sich Abschnitte des Hochtemperaturbereichs mit Abschnitten des Niedertemperaturbereichs entlang des ersten Kanals (101) abwechseln und die Bereiche (103a, 103b) voneinander getrennt sind.

2. Vorrichtung (100) nach Anspruch 1, wobei ein erster Temperaturbereich geeignet ist, um eine Temperatur bereitzustellen, die ausreicht, um zu einer Denaturierung doppelsträngiger Nukleinsäuren zu führen, um einzelsträngige Nukleinsäuren herzustellen, ein zweiter Temperaturbereich geeignet ist, um eine Temperatur bereitzustellen, die ausreicht, um zu einer Hybridisierung von Primern an die einzelsträngigen Nukleinsäuren zu führen, und ein dritter Temperaturbereich geeignet ist, um eine Temperatur bereitzustellen, die ausreicht, um zu einer Amplifikation einzelsträngiger Nukleinsäuren zu führen, um doppelsträngige Nukleinsäuren herzustellen.

3. Vorrichtung (100) nach Anspruch 1, ferner umfassend einen zweiten Kanal, wobei der zweite Kanal an einer Oberseite offen ist und der erste Kanal (101) innerhalb des zweiten Kanals liegt.

4. Vorrichtung (100) nach Anspruch 1, wobei die Vorrichtung (100) mit einem Anregungssystem gekoppelt ist.

5. Vorrichtung (100) nach Anspruch 4, wobei die Vorrichtung (100) mit einer optischen Erfassungsvorrichtung gekoppelt ist.

6. Vorrichtung (100) nach Anspruch 5, wobei die optische Erfassungsvorrichtung ein Spektrograph ist.

7. Vorrichtung (100) nach Anspruch 1, wobei die Vorrichtung mit einem Flüssigkeitsbrückensystem fluidisch gekoppelt ist.

8. Vorrichtung (100) nach Anspruch 1, wobei die Tröpfchen in einem nicht mischbaren Fluid eingehüllt sind.

9. Vorrichtung (100) nach Anspruch 1, wobei die Nukleinsäure DNA oder RNA ist.

10. Verfahren zum Durchführen einer quantitativen Polymerasekettenreaktion unter Verwendung einer Vorrichtung (100) nach einem der Ansprüche 1-9, wobei das Verfahren die folgenden Schritte umfasst:
a) Fließen von Probentröpfchen, die markierte Nukleinsäuren umfassen, durch den wenigstens einen ersten Kanal (101), wobei die markierten Nukleinsäuren in den Tröpfchen optisch erfassbar sind, während die Tröpfchen durch den ersten Kanal (101) fließen;
b) Denaturieren von doppelsträngigen Nukleinsäuren in den fließenden Probentröpfchen, um einzelsträngige Nukleinsäuren herzustellen;
c) Hybridisieren von Primern an die einzelsträngigen Nukleinsäuren in den fließenden Probentröpfchen;
d) gleichzeitiges Amplifizieren der Nukleinsäuren und Erfassen der amplifizierten Nukleinsäuren in den fließenden Tröpfchen; und
e) Wiederholen der Schritte (b) bis (d) wenigstens einmal.

11. Verfahren nach Anspruch 10, wobei die Tröpfchen in einem nicht mischbaren Fluid eingehüllt sind.

12. Verfahren nach Anspruch 10, wobei das Verfahren vor Schritt (a) ferner ein Ausbilden von Probentröpfchen unter Verwendung eines Flüssigkeitsbrückensystems umfasst.

13. Verfahren nach Anspruch 10, wobei der erste Kanal (101) innerhalb eines zweiten Kanals liegt, der an einer Oberseite offen ist.

## Revendications

1. Dispositif (100) destiné à amplifier des acides nucléiques, le dispositif (100) comprenant :
au moins un premier canal (101) à travers lequel s'écoulent des gouttelettes d'échantillon comprenant des acides nucléiques, les acides nucléiques présents dans les gouttelettes étant détectables optiquement pendant que les gouttelettes s'écoulent à travers le premier canal (101) ; et
une pluralité de zones de température comprenant une zone à haute température (103a) et une zone à basse température (103b), chaque zone ayant un profil défini par des saillies rectangulaires alternant avec des évidements rectangulaires, chaque évidement rectangulaire de la zone à haute température entourant une saillie rectangulaire correspondante de la zone à basse température et inversement, la zone à haute température et la zone à basse température étant isolées thermiquement l'une de l'autre par des entrefers (104), la zone à haute température (103a) et une zone à basse température (103b) étant en contact thermique avec le premier canal (101), qui est conçu en ligne droite dans la zone de contact thermique avec la zone à haute et la zone à basse température, des parties de la zone à haute température alternant avec des parties de la zone à basse température le long du premier canal (101) et les zones (103a, 103b) étant séparées l'une de l'autre.

2. Dispositif (100) selon la revendication 1, dans lequel une première zone de température est appropriée pour fournir une température suffisante pour obtenir une dénaturation des acides nucléiques bicaténaires afin de produire des acides nucléiques monocaténaires, une deuxième zone de température est appropriée pour fournir une température suffisante pour obtenir l'hybridation d'amorces avec les acides nucléiques monocaténaires, et une troisième zone de température est appropriée pour fournir une température suffisante pour obtenir une amplification des acides nucléiques monocaténaires afin de produire des acides nucléiques bicaténaires.

3. Dispositif (100) selon la revendication 1, comprenant en outre un second canal, le second canal étant ouvert en haut et le premier canal (101) se trouvant à l'intérieur du second canal.

4. Dispositif (100) selon la revendication 1, dans lequel le dispositif (100) est couplé à un système d'excitation.

5. Dispositif (100) selon la revendication 4, dans lequel le dispositif (100) est couplé à un dispositif de détection optique.

6. Dispositif (100) selon la revendication 5, dans lequel le dispositif de détection optique est un spectrographe.

7. Dispositif (100) selon la revendication 1, dans lequel le dispositif est couplé de manière fluidique à un système de ponts liquides.

8. Dispositif (100) selon la revendication 1, dans lequel les gouttelettes sont enveloppées dans un fluide immiscible.

9. Dispositif (100) selon la revendication 1, dans lequel l'acide nucléique est l'ADN ou l'ARN.

10. Procédé d'exécution d'une réaction en chaîne par polymérase quantitative à l'aide d'un dispositif (100) selon l'une quelconque des revendications 1 à 9, le procédé comprenant les étapes consistant à :
a) faire s'écouler des goutelettes d'échantillon comprenant des acides nucléiques étiquetés à travers l'au moins un premier canal (101), les acides nucléiques étiquetés dans les gouttelettes étant détectables optiquement pendant que les gouttelettes s'écoulent à travers le premier canal (101) ;
b) dénaturer des acides nucléiques bicaténaires dans les gouttelettes d'écoulement de l'échantillon pour produire des acides nucléiques monocaténaires ;
c) hybrider des amorces aux acides nucléiques monocaténaire dans les gouttelettes d'écoulement de l'échantillon ;
d) amplifier simultanément les acides nucléiques et détecter les acides nucléiques amplifiés dans les gouttelettes d'écoulement ; et
e) répéter les étapes (b) à (d) au moins une fois.

11. Procédé selon la revendication 10, selon lequel les gouttelettes sont enveloppées dans un fluide immiscible.

12. Procédé selon la revendication 10, selon lequel avant l'étape (a), le procédé comprend en outre la formation de gouttelettes d'échantillon à l'aide d'un système de ponts liquides.

13. Procédé selon la revendication 10, selon lequel le premier canal (101) se trouve à l'intérieur d'un second canal ouvert en haut.
